# EUROPEAN PATENT APPLICATION

(11) **EP 2 113 256 A1**
(43) Date of publication of application: **04.11.2009**
(21) Application number: 08155408.1
(22) Date of filing: 29.04.2008
(51) Int. Cl.: A61K 47/48, A61P 43/00

(54) **PEGylated rhGH compounds**

(71) Applicant: Ascendis Pharma AS, 2900 Hellerup (DK)
(72) Inventor: Sprogøe, Kennett, DK-2900, Hellerup (DK)
(74) Representative: Büchel, Edwin

(57) **Abstract**

A chemically modified human Growth Hormone (rhGH) prepared by attaching a transient linker which comprises a polyethylene glycol. The chemically modified protein may have a much longer lasting rhGH activity than that of the unmodified rhGH, enabling reduced dose and scheduling opportunities and the modified rhGH may not cause lipoatrophy. Also includes methods of use for the treatment and/or prevention of diseases or disorders in which use of growth hormone is beneficial.

## Description

### FIELD OF THE INVENTION

This invention relates to relates to a pharmaceutical composition comprising suitable pharmaceutical excipients and also comprising a human *in vivo* clinical effective amount of a recombinant human growth hormone (rhGH) PEGylated prodrug which may not cause injection side lipoatrophy.

### BACKGROUND ART

Growth hormone (GH) is a hormone that stimulates growth and cell reproduction in humans and other animals. It is a 191-amino acid, single chain polypeptide hormone which is synthesized, stored, and secreted by the somatotroph cells within the lateral wings of the anterior pituitary gland. The hormone is also known as somatotrophin when referring to growth hormone produced by recombinant DNA technology, and is abbreviated "rhGH".

Growth hormone has a variety of functions in the body, the most noticeable of which is the increase of height throughout childhood, and there are several diseases which can be treated through the therapeutic use of GH.

The effects of growth hormone deficiency vary depending on the age at which they occur. In children, growth failure and short stature are the major manifestations of GH deficiency. It can also cause sexual immaturity. In adults the effects of deficiency are more subtle, and may include deficiencies of strength, energy, and bone mass, as well as increased cardiovascular risk.

There are many causes of GH deficiency, including mutations of specific genes, congenital malformations involving the hypothalamus and/or pituitary gland, and damage to the pituitary from injury, surgery or disease.

Deficiency is treated through supplementation with external GH. All GH in current use is a biosynthetic version of human GH, manufactured by recombinant DNA technology. As GH is a large protein molecule, it must be injected subcutaneously to access the bloodstream (injections no longer have to enter muscle mass since 1985 with the production of synthetic GH). GH is used as replacement therapy in children and adults with GH deficiency of either childhood-onset (after completing growth phase) or adult-onset (usually as a result of an acquired pituitary tumor). In these patients, benefits have variably included reduced fat mass, increased lean mass, increased bone density, improved lipid profile, reduced cardiovascular risk factors, and improved psychosocial well-being.

Genentech Inc (US) was the first to clone rhGH around 1980. As of 2005, synthetic growth hormones available in the United States and Europe (and their manufacturers) included Nutropin (Genentech), Humatrope (Lilly), Genotropin (Pfizer), Norditropin (Novo), and Saizen (Merck Serono). In 2006, the U.S. Food and Drug Association (FDA) approved a version of rhGH called Omnitrope (Sandoz).

Although molecular biological techniques have dramatically increased the availability of many proteins and/or polypeptides (hereinafter referred to as proteins), the therapeutic use of said proteins is often times hindered by other factors, such as aggregation, proteolytic degradation, poor bioavailability and physical properties which preclude efficient formulations.

A mechanism for enhancing protein availability is by conjugation of the protein with derivatizing compounds, which include, but are not limited to, polyethylene glycol and polypropylene glycol. Some of these benefits recognized include: lowered immunogenicity and antigenicity, increased duration of action, and altered pharmacokinetic properties. [Veronese, F.M. "Enzymes for Human Therapy: Surface Structure Modifications," Chimica Oggi, 7:53-56 (1989)] (Herein reference 5).

The PEGylated rhGH may increase plasma residency duration, decrease clearance rate (hereby achieving reduced dosage), improve stability and decrease antigenicity or a combination thereof.

Generally, this is well known in the art and there are numerous patent applications available in the patent literature. For example a PEGylated Erythropoietin (EPO) from Hofmann La Roche described in EP119644B1 claiming a specific linker comprising PEG covalently bound to EPO, a PEGylated interferon alpha described in EP975369B1 from the company Nektar/La Roche and another PEGylated interferon alpha in EP1562634B1 from the company Hofmann La Roche.

Clearance mechanism is believed to occur by following two mechanisms. The first is renal where rhGH is cleared from the circulation by renal mechanisms. Renal clearance of rhGH is well documented and PEGylation of synthetic rhGH is therefore an obvious choice to solve this problem. Renal clearance accounts for around 25 - 53 % of the total clearance of rhGH (Girard, J. Mehls, O.J. Clin. Invest. 1994 March; 93(3): 1163-1171, reference 3 herein.)

The second mechanism is hepatic clearance (liver). Hepatic GH uptake occurs by receptor-mediated endocytosis followed by lysosomal degradation. By reducing receptor recognition by PEGylation, the hepatic clearance will be reduced.

However, there are dedicated problems with the PEGylation conjugates of rhGH.

One major disadvantage of subcutaneously administrated PEGylated rhGH is the phenomenon of lipoatrophy.

Lipoatrophy is the medical term used for fat loss. Known PEGylated rhGH compounds have displayed lipoatrophy problems, which is believed due to residual activity of the growth hormone complex and the increase residence time near the injection site. The term "residual activity" is understood herein as the activity of the drug-polymer conjugate and is governed by the PEGylation site and the nature of the PEG molecule.

Büyükgebiz A. et al published in J. Pediatr. Endocrinol. Metab. 1999 Jan-Feb; 12(1):95-7 describes such a medical record (herein reference 1). This is a report of a patient with isolated GH deficiency due to 6.7 kb gene deletion who received high dose rhGH treatment and developed local lipoatrophies at injection sites without any antibody detection after 6 years of therapy. The etiology of the lipoatrophy is suspected to be by the direct lipolytic effect of high doses of rhGH.

Lipoatrophy related to the administration of rhGH is believed to be caused by the rhGH activity itself, by higher concentrations and by prolonged exposure. These higher concentrations occur near injections sites.

The chance that high growth hormone activity accumulates near the injection site is even higher in case that rhGH is PEGylated. In the case of PEGylated rhGH formulations, the tissue will experience a sustained and increased exposure to growth hormone activity, due to the fact that the PEGylated conjugate possess residual activity necessary for pharmacological activity and the conjugate is diffusion limited due to the conjugate size. In other words, the PEGylated rhGH is not totally inactive. The outcome is increased lipolysis at the injection site.

An example of such a PEGylated hGH compound, which exhibits lipoatrophy, is the compound PHA-794428. Compound PHA-794428 is a PEGylated rhGH and described in patent EP1715887 from the company Pharmacia (acquired by Pfizer) and further described in Horm. Res. 2006; 65 (suppl. 4): 1-213, CF1-98 GH/IGF Treatment with title "First in-human study of PEGylated recombinant human growth hormone", Philip Harris et al. (herein reference 4)

According to the clinical trial information as published on www.clinicaltrials.gov, the trial was terminated on 10-Dec-2007. Pfizer's (above referred Pharmacia EP1715887) decision to terminate the program was due to cases of injection-site lipoatrophy that were reported in the clinical Phase 2 studies after a single injection of PHA 794428.

W02006102659 (Nektar) also describes and suggests rhGH-PEG conjugates (linear and branched types). The problem to be solved in application W02006102659 is described in paragraph [0005] page 2. As the applicant describes it, the problem to be solved is reduced dosing frequency. Since rhGH therapy typically requires daily injections, patients, and in particular, pediatric patients, dislike the inconvenience and discomfort associated with this regimen. The solution described in Nektar application is new PEG-rhGH conjugates.
In table 6, [0257] can be seen that the PEG-rhGH conjugates has a relatively low acticity *in vitro* as compared to the native growth hormone without PEG. The PEG-rhGH conjugates were tested *in vivo* and to their surprise they found that the *in vitro* inactive PEG-rhGH conjugates worked fine *in vivo* - i.e. giving a weight gain corresponding to rhGH as such (without PEG). In relation to this reads section [0261]:
"Although the preliminary *in vitro* results suggest that increasing the amount of PEG attached to hGH reduces its ability to stimulate the hGH receptor, based on the preliminary *in vivo* results, it appears that a reduction in bioactivity is more than balanced by increased half-life and/or plasma availability, thus leading to a conclusion that the conjugates provided herein possess a superior pharmacodynamic effect *in vivo* when compared to unmodified rhGH at an identical dosing regimen".

W02006102659 (Nektar) does not describe anything about a cleavable linker- i.e. it is simple said that PEG-rhGH works as such *in vivo* even though it is inactive *in vitro*. Accordingly, one may objectively say that this document teaches away from using a cleavable linker such as a self hydrolysable (autocleavage) transient linker.
Further in this document is not mentioned anything about any possible lipoatrophy problems.

The company "Complex Biosystems GmbH" produced relevant prior art (W02005099768) in the field of PEGylated linker molecules mentioning herein rhGH (claim 6). In application W02005099768, the problem to be solved is the interpatient variability and unpredictable effect of prodrug activation when enzymatic mechanism is involved (page 12, line 17-30). This application describes as a solution an aromatic linker (PEG based). This linker-PEG binds the drug in a way that the drug activity is significantly reduced. It is activated only on release of the drug, which is initiated by hydrolysis. The hydrolysis rate can be controlled chemically.

In W02005099768 is GH simple mentioned in a long list of possible relevant biologics and in the working examples is the biologic insulin. Accordingly, in this document there is no special emphasis on GH and relevant problems in relation to this as such.

In summary, none of the above mentioned citations efficiently solve the problem of lipoatrophy related to the use of PEGylated-rhGH compounds.

### SUMMARY OF THE INVENTION

The problem to solve in present invention is to reduce the administration frequency of rhGH using PEG conjugated to rhGH without significantly inducing lipoatrophy.

Today patients suffering from growth hormone deficiency need daily injections.

Lipoatrophy is the medical term used for fat loss. As said above, known PEGylated rhGH compounds have displayed lipoatrophy problems, which is believed due to residual activity of the growth hormone complex and the increase residence time near the injection site.

Herein further described are relevant situations where the problem of lipoatrophy can occur.

In a first situation is the injection of native rhGH. In this case rhGH is present subcutaneously with full biological activity. As said above, subcutaneous administrated rhGH can cause lipoatrophy as reference to Büyükgebiz A. et al published in J. Pediatr. Endocrinol. Metab. 1999 Jan-Feb; 12(1):95-7 and see also the prescribing information of a synthetic rhGH, Norditropin from the company Novo Nordisk A/S. As described in the prescription document reads on line 2.3, lipoatrophy is avoided by the rotation of the injection sites.

A second situation is the administration of permanently PEGylated rhGH. The conjugate will have reduced biological activity due to the conjugated PEG molecule. Due to the necessity of preserving enough residual biological activity for eliciting the desired pharmacological response, the conjugate will be able to induce lipoatrophy. This is exacerbated by the prolonged tissue exposure caused by reduced diffusion of PEG-rhGH conjugate from the injection site. (an example of such situation is demonstrated in reference compound PHA-794428 discussed in www.clinicaltrials.gov related to lipoatrophy.

The third situation is the administration of transiently PEG conjugated rhGH.

In a situation, where the residual activity of the prodrug (as is the case for transiently PEG conjugated rhGH) is inactive, lipolytic effects will not occur even at prolonged exposure.

As said above, rhGH as such or available commercially known under trade name; Nutropin ® (see also Nutropin prescribing information on www.nutropin.com/patient) exhibit lipolytic effects. Lipolytic effects also occur with straight forward PEGylated rhGH, such as compound PHA-794428. This is because the PEGylated drug has a remaining residual activity in order to exhibit the biological activity. PEGylated drugs always carry the covalently bound PEG attached to the drug.

Also as said above W006102659 (Nektar), describes PEG conjugated rhGH. The therein suggested PEG conjugates rhGH exhibit reduced activity in vitro (see table 6 page 78 e.g. structure specimen 1 D activity 0.4% and specimen 3M activity of 11.4%, see table 6 page 78). As further described by the inventors (paragraph 258) of application W006102659, *in vitro* results suggest that increasing the amount of PEG attached to hGH reduces its ability to stimulate the hGH receptor. Based on the preliminary *in vivo* results, it appears that a reduction in bioactivity is more than balanced by increased half-life and/or plasma availability, thus leading to a conclusion that the conjugates provided therein possess a superior pharmacodynamic effect *in vivo* when compared to unmodified rhGH at an identical dosing regimen. However, the inventors in application W006102659 are silent about the negative side effects such as lipoatrophy.

As discussed above, W02006102659 (Nektar) does not describe anything about a cleavable linker- i.e. it is simple said that PEG-rhGH works as such *in vivo* even though it is inactive *in vitro*. Accordingly, one may objectively say that this document teaches away from using a cleavable linker such as a self hydrolysable (autocleavage) transient linker.

The herein described compounds termed transiently PEGylated prodrugs are compounds which are also inactivated. To activate the drugs, the described compounds herein need to undergo an activation step (1,6 release mechanisms), termed herein as autocleavage, releasing the PEG group from the drug. The 1,6 release mechanism is well described in W005099768 (page 8 line 25).

Without being limited to theory, the present inventors believe that the herein disclosed transiently PEGylated rhGH compounds significantly reduce lipoatrophy. The theory is that the relatively low activity of the PEGylated rhGH compounds, before PEG is gradually cleaved off by the auto cleavable linker, do that one gets a significantly reduction of lipoatrophy.

Further, compared to W02006102659 (Nektar) it is an advantage that PEGylated rhGH compounds as described herein comprise a self hydrolysable (autocleavage) transient linker that gradually cleave off PEG to get rhGH without PEG. One hereby gradually in a controlled way get "full" active rhGH and one can therefore use a smaller initial dose - as compared to W02006102659 (Nektar).

Thus one can solve this lipoatrophy problem by the reduction of residual activity as measured *in vitro* of the transient PEGylated compounds as disclosed herein.

As discussed above, a PEGylated prodrug, wherein the drug is for example rhGH as described in patent application W02005099768, and has a characteristic of release, which is therein described as the 1,6 cleavage system without the production of toxic aromatic compounds. In this document is generically/broadly described numerous herein relevant suitable so-called transient linker structures to get a relevant release profile of interest.

Other "transient" linker structures are generically/broadly described in e.g. other Complex Biosystems GmbH applications such as W005034909, W005099768, W006003014 and W02006136586.

Further in these application the numerous listed biologics may be linked to numerous different polymers such as human serum albumin, dextran, lectins, poly (styrene-comaleic anhydride), poly(N-hydroxypropylmethacrylamide), poly(divinyl ether-comaleic anhydride), hyaluronic acid and PEG may be seen as an example of one of these numerous different polymers.

In other to solve the present problems for GH as discussed herein one may say that the present inventors have selected suitable preferred transient linker structures to get the herein described relevant functional properties of the rhGH PEGylated prodrug. Based on the herein detailed description of preferred linkers structures it is within the skilled person knowledge to make other suitable preferred transient linker structures that could give an rhGH PEGylated prodrug with the herein described relevant functional properties.

The herein relevant functional properties of the rhGH PEGylated prodrug are:
(1): when PEG is linked to rhGH in the prodrug conjugate the prodrug has an GH activity with is less than 5% of the native growth hormone without PEG to avoid injection side lipoatrophy; and
(2): PEG is linked to rhGH via a self hydrolysable (autocleavage) transient linker, wherein the linker autohydrolysis rate is such that the *in vivo* half-life is from 10 hours to 600 hours.

Property (1) gives a very low lipoatrophy negative side-effect. Without being limited to theory the present believes are that if the PEG-rhGH product as such has a higher GH activity as such then this product would still have a clinically too high lipoatrophy negative side-effect.

Property (2) ensures that rhGH (without PEG) is released gradually over time so one can administrate the rhGH pharmaceutical product e.g. only once weekly or monthly and after e.g. a week or month, still have sufficient *in vivo* amounts of active rhGH to ensure suitable rhGH coverage.

Accordingly, a first aspect of the invention relates to a pharmaceutical composition comprising suitable pharmaceutical excipients and also comprising a human *in vivo* clinical effective amount of a recombinant human growth hormone (rhGH) PEGylated prodrug conjugate; wherein the prodrug is characterized by that:
(1): when PEG is linked to rhGH in the conjugate the conjugate has an GH activity which is less than 5% of the native growth hormone without PEG, measured according to the assay to measure rhGH PEGylated prodrug and rhGH activity of example 1; and
(2): PEG is linked to rhGH via a self hydrolysable (autocleavage) transient linker, wherein the linker autohydrolysis rate is such that the in vivo half-life range from 10 hours to 600 hours, measured according to the assay to measure autocleavage rate of the transient linker of the prodrug of example 2.

Accordingly, a second aspect of the invention relates to a clinical effective amount of the pharmaceutical composition comprising the rhGH PEGylated prodrug of the first aspect for use in a method for treatment of a GH related disease in a human person.

This second aspect may alternatively be formulated as a method for treatment of a GH related disease in a human person comprising administrating to a human person a clinical effective amount of the pharmaceutical composition comprising the rhGH PEGylated prodrug of the first aspect.

Embodiment of the present invention is described below, by way of examples only.

### DEFINITIONS

Prior to a discussion of the detailed embodiments of the invention is provided a definition of specific terms related to the main aspects of the invention.

In general, all specific technical terms used herein shall be understood as the skilled person would understand them in the present technical context.

The term "activity" herein is understood as the ability of growth hormone or a conjugate thereof, to evoke a biological response when administered to a mammal, e.g. in an *in vivo* model, or to produce a measureable response in *an in vitro* model as described in examples.

The term "autocleavage" herein is understood as spontaneous cleavage of the bond between the transient linker and the drug molecule rhGH under physiological conditions.

The term "conjugate" herein is understood as a PEG molecule covalently bound to the drug herein being human growth hormone.

The term "*in vivo* half life" is understood as the time interval in which 50% of the initial proportion of the growth hormone is released from the conjugate after administration to the human body.

The term "Lipoatrophy" herein is understood as a medical term for fat loss. In the present context "lipoatrophy" refers to injection site lipoatrophy meaning lipolysis occurring in close proximity of the injection site.

The term "Prodrug" herein is understood is any compound that undergoes biotransformation before exhibiting its pharmacological effects. Prodrugs can thus be viewed as drugs containing specialized non-toxic protective groups used in a transient manner to alter or to eliminate undesirable properties in the parent molecule.

The term a pharmaceutical composition comprising "a human *in vivo* clinical effective amount of a recombinant human growth hormone (rhGH) PEGylated prodrug" is to be understood as an amount that is sufficiently high to obtain a wanted clinical effect in a human after administration of the pharmaceutical composition to the human - e.g. a wanted clinical effect in relation to treatment of a GH related disease. In the present context the skilled person routinely is able to adjust the amount of recombinant human growth hormone (rhGH) PEGylated prodrug to be administrated in order to get a wanted clinical effect.

The term "physiological condition" herein is understood as any *in vitro* or *in vivo* condition, identical or resembling, the conditions in the human body. More specifically physiological conditions is referring to conditions at around pH 7.4 (pH 6.8 to pH 7.8) and about 37°C (35°C to 40°C).

The term "transient" linker is a linker in which the conjugation of drug to PEG molecule is reversible. This implies that autocleavage of the linker releases the drug in its native and active form. Furthermore, cleavage of the linker proceeds at a predictable manner releasing the unmodified drug over many hours independent of enzyme activity.

### DETAILED DESCRIPTION OF THE INVENTION

### Pharmaceutical composition comprising suitable pharmaceutical excipients

As known to the skilled person a pharmaceutical composition comprises pharmaceutical acceptable excipients and/or carriers.

"Pharmaceutically acceptable" is meant to encompass any excipient and/or carrier, which does not interfere with the effectiveness of the biological activity of the active ingredient and that, is not toxic to the host to which it is administered.

In a preferred embodiment the pharmaceutical composition is a composition for subcutaneous administration, intramuscular administration or intravenous injection.
This is examples of preferred administration routes for treatment of a relevant disorder/disease as described herein.

The pharmaceutical composition may comprise other active ingredients than a rhGH PEGylated prodrug as described herein.

### Recombinant human growth hormone (rhGH)

Beside that the recombinant human GH is identical in sequence to natural human GH, the term recombinant human growth hormone (rhGH) relates herein also to so-called biogenerics equivalent.

The term "biogenerics" herein is understood generic forms of biopharmaceuticals; molecules developed using biological processes, usually through modern biotechnology activity. Generic chemical pharmaceuticals can be defined as those molecules which, when compared with the originator product: have essentially similar activity, are essentially chemically identical to their branded counterparts, are bioequivalent, achieve market authorization through an abbreviated procedure following patent expiry.

As known to the skilled person, it is today routine work to make e.g. minor amino changes of a biologics of interest (herein GH) without significantly affecting the activity of the biologics.

### rhGH PEGylated prodrug - preferred PEG

As discussed above, the rhGH PEGylated prodrug as described herein shall have a relatively low activity.

Accordingly, in a preferred embodiment the total PEG load per growth hormone molecule amounts to at least 25 kDa. Generally the PEG load will be less than 1000 kDa.

The PEG chain or chains may be branched or linear and comprise one or more PEG chains per conjugate. A branched PEG is a PEG molecule consisting of a branching point connecting two or more PEG chains, to form a molecule with one anchoring point for attachment to growth hormone. This could be two 20 kDa PEG chains joined to form one branched 40 kDa PEG molecule. In the case where the molecule contains two or three branching points, the molecule is referred to 3 and 4 armed PEG, respectively.

In summary, the PEG polymer is not limited to a particular structure and can be linear, branched, or multi-armed (e.g. forked PEG or PEG attached to a polyol core), dendritic, or with degradable linkers.

Without being limited to theory such a size of PEG is a suitable size to get the required relatively low activity and not having a too big size of the PEG that could create other problems.

The PEGylation to native human GH may occur on several lysine groups or on the terminal amine (F1) as well described by Clark et al. (reference 2 herein) on page 21973 table III. As highly reactive is position F1 and LYS-140. Moderate reactive is LYS-115, 38, and 70. Poorly reactive are positions LYS-172, 41, 158 and 168.

### rhGH PEGylated prodrug - "transient" linker structures

As discussed above, a PEGylated-prodrug, wherein the drug is for example rhGH as described in patent application W02005099768, and has a characteristic of release, which is therein described as the 1,6 cleavage system without the production of toxic aromatic compounds. In this document is generically/broadly described numerous herein relevant suitable so-called transient linker structures to get a relevant release profile of interest. Other "transient" linker structures are generically/broadly described in e.g. other Complex Biosystems GmbH applications such as W005034909, W005099768, W006003014 and W02006136586.

More "transient" linker structures are generically/broadly described in e.g. W09930727 (Enzon Inc).

In order to solve the present problems for GH as discussed herein one may say that the present inventors have selected suitable preferred transient linker structures to get the herein described relevant functional properties of the rhGH PEGylated prodrug. Based on the herein detailed description of preferred linkers structures it is within the skilled person knowledge to make other suitable preferred transient linker structures that could give an rhGH PEGylated prodrug with the herein described relevant functional properties.

The herein selected linker structures are described in detail below.

Ideally, a conjugate of the invention will possess one or more of the following features and/or advantages over current rhGH conjugates or formulations; can easily be synthesized in good yields, have half life's falling within preferred range, can be purified to provide homogeneous conjugate compositions, exhibit activity after autocleavage such as *in vitro* and *in vivo* activity and have pharmacodynamic effects superior to unmodified rhGH and previously described rhGH conjugates and do not cause lipoatrophy.

The herein described structures exhibit release properties as required herein.

Preferred general aromatic linker structures are listed below. 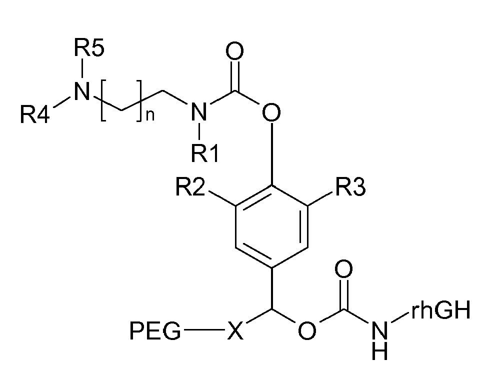 R1, R2, R3, R4, and R5 are selected independently from hydrogen, methyl, ethyl, propyl, isopropyl, butyl, isobutyl, tertiary butyl,
and n = 1 or 2, and

X is selected from C1 to C8 alkyl or C1 to C12 heteroalkyl.

More preferred structures of general formula I, which a part of the structure within the general aromatic linker structure above: 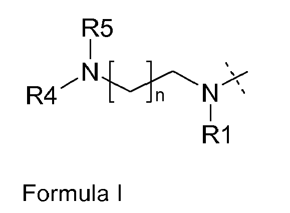 and where preferred examples of formula I comprise:

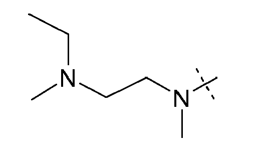

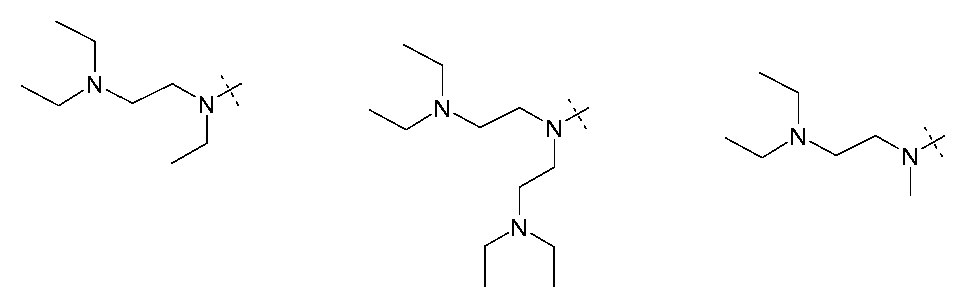

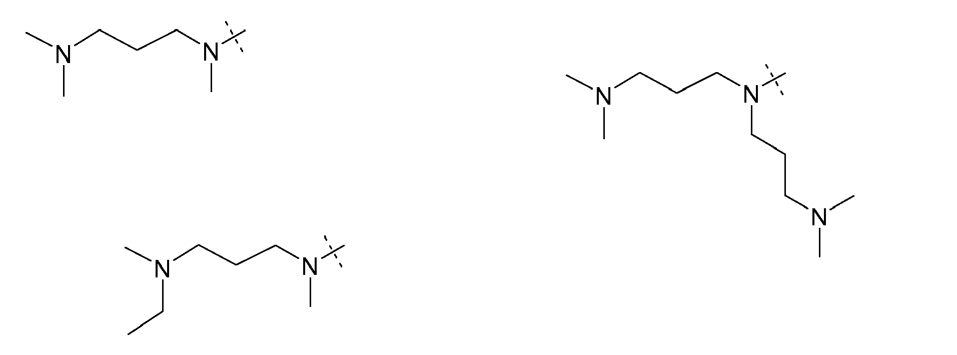

More preferred aromatic structures of formula II, which a part of the structure within the general aromatic linker structure above: 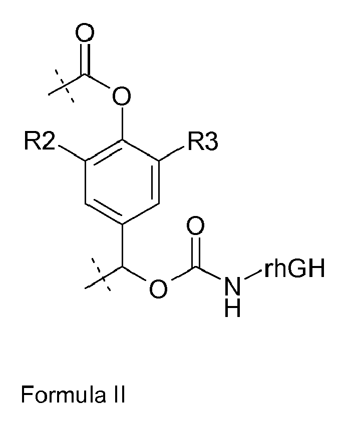 and where preferred examples of formula II comprise:

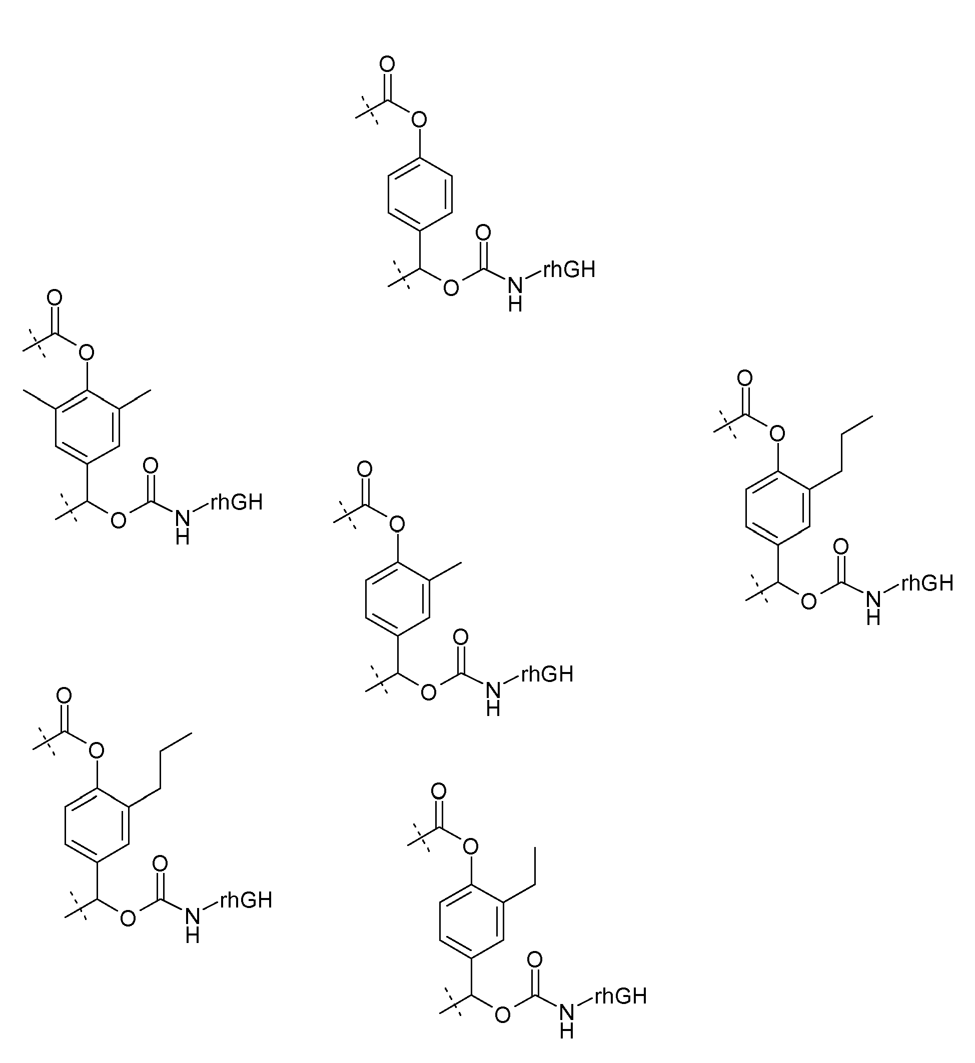

More preferred structures of formula III, which a part of the structure within the general aromatic linker structure above: 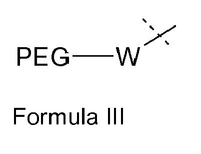 and PEG-W includes the following substituent groups:

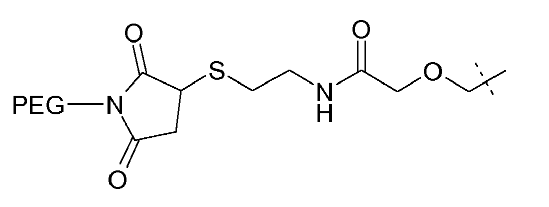

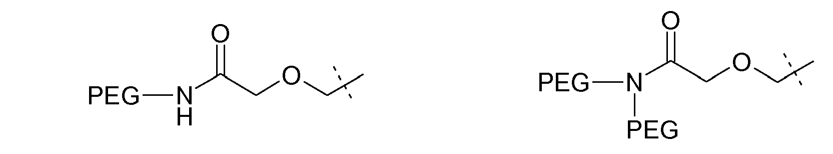

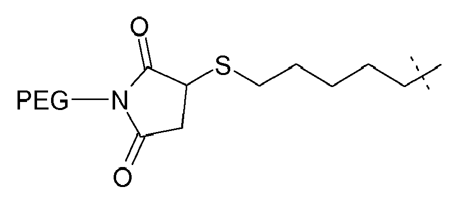

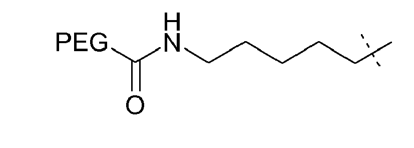

One example of preferred self hydrolysable transient linker:

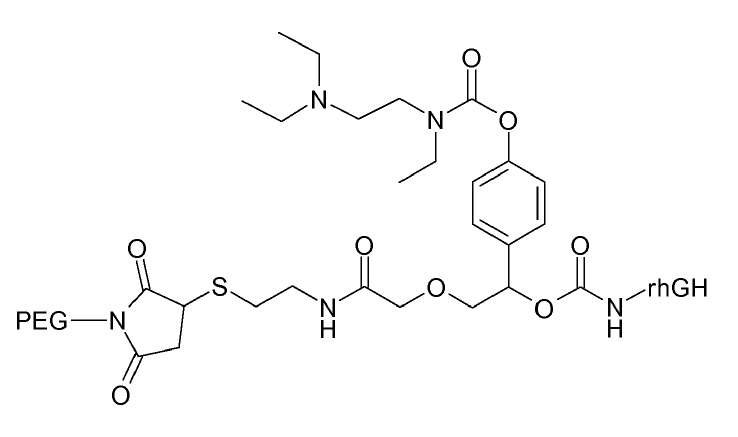

Other preferred self hydrolysable transient linker structures are: 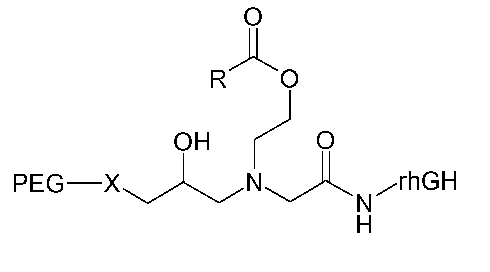 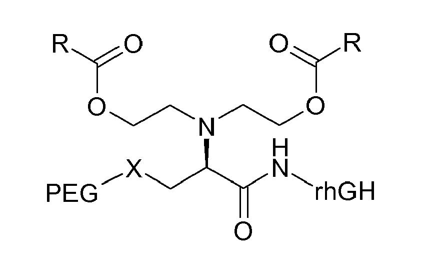 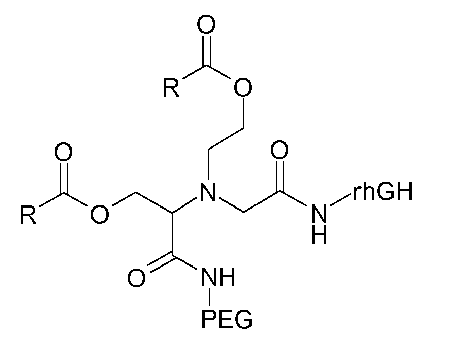 R is selected from hydrogen, methyl, ethyl, propyl and butyl,
X is selected from C1 to C8 alkyl or C1 to C12 heteroalkyl.

Assays to determine the functional properties of rhGH PEGylated prodrug

As said above the rhGH PEGylated prodrug as described herein is characterized by that:
(1): when PEG is linked to rhGH in the prodrug conjugate the prodrug has an GH activity with is less than 5% of the native growth hormone without PEG to avoid injection side lipoatrophy; and
(2): PEG is linked to rhGH via a self hydrolysable (autocleavage) transient linker, wherein the linker autohydrolysis rate is such that the *in vivo* half-life is from 10 hours to 600 hours.

The assay to determine property (1) is described in details in working example 1 herein. Based on these detailed instructions it is routine work for the skilled person to measure this residual activity of the prodrug.

In a preferred embodiment the residual activity of property (I) is less than 5%, more preferably less than 3%, even more preferably less than 1% and most preferably virtually inactive.

The assay to determine property (2) is described in details in working example 2 herein. Based on these detailed instructions it is routine work for the skilled person to measure this autocleavage rate of the transient linker of the prodrug.

In a preferred embodiment the autocleavage rate *in vivo* half-life is such that the *in vivo* half-life is from 20 hours to 300 hours, more preferably from 30 hours to 150 hours, even more preferably from 40 hours to 100 hours and most preferably from 50 to 75 hours.

### Lipoatrophy

The assay to determine lipoatrophy is described in details in working example 3 herein. Based on these detailed instructions it is routine work for the skilled person to measure lipoatrophy.

In a preferred embodiment, the rhGH PEGylated prodrug conjugate, as described herein, has a lipoatrophy effect of less than 75% of the corresponding rhGH without PEG, measured according to the assay to determine lipoatrophy of example 3 and other identical dosage regimen conditions.
More preferably rhGH PEGylated prodrug conjugate has a lipoatrophy effect of less than 20 % of the corresponding rhGH without PEG, even more preferably the rhGH PEGylated prodrug conjugate has a lipoatrophy effect of less than 10% of the corresponding rhGH without PEG and most preferably the rhGH PEGylated prodrug conjugate has a lipoatrophy effect of less than 5% of the corresponding rhGH without PEG.

### GH related disease

The term "a GH related" disease of second aspect simply herein relates to diseases and conditions where a human could benefit from GH.

This includes, but is not limited to, growth hormone deficiency, adult onset growth hormone deficiency, Turner syndrome, Prader-Willi syndrome, short bowel syndrome, chronic renal insufficiency, small for gestational age, AIDS wasting, anti-ageing, rheumatoid arthritis, idiopathic small stature, short stature homeobox gene and somatopause. Included is also other short stature condition, which includes Noonan syndrome, skeletal dysplasia, Down syndrome, short stature associated with prolonged steroid use, Aarskog's syndrome, among others.

### EXAMPLES

### Example 1: Assay to measure rhGH PEGylated prodrug and rhGH activity

The biological activity of rhGH is measured by using standard assays known to the skilled person in the art. As described in EP1715887B1 and as also discussed above, the biological activity associated with the native or unmodified hGH (for example a PEGylated rhGH), as measured using standard FDC-P1 cell proliferation assays, (Clark et al, Journal of Biological Chemistry 271: 21969-21977), receptor binding assay (US5057417), or hypophysectomized rat growth (Clark et al, Journal 271: 21969-21977, 1996). On line 8 (page 14) of patent EP1715887B1, it is described that the preferred in vitro activity has to be as high as possible, most preferred the modified rhGH has equivalent or improved in vitro biological activity. In current invention, the biological activity has to be as little as possible compared to native rhGH. Thus current inventors did the complete opposite compared to the prior art described in EP1715887B1.

### In vitro Assay

The *in vitro* activities of the (rhGH)-PEG conjugates described in the preceding Examples are determined using one or more standard assays for assessing biological activity *in vitro*. Standard assays that may be employed include cell proliferation assays using, e.g., FDC-PI cells (see, e.g., Clark et al., Journal of Biological Chemistry, 271:21969-21977, 1996), or Ba/F3-hGHR cells, which express receptors for hGH, hGH delta 135-146, or Nb2 rat lymphoma cells, which proliferate in response to hGH via the lactogenic receptors (see, e.g., Alam, K. S., et al., J. Biotech 2000, Feb. 28, 78(1), 49-59). Receptor binding assays (see, e.g., U.S. Pat. No. 5,057,417), and hypophysectomized rat growth (Clark, et al., Journal of Biological Chemistry, ibid) may also be used.

Nb2-11 is a clone of the Nb-2 rat lymphoma line which was derived from a transplant of a lymphoma that developed in the thymus/lymph node of a male noble (Nb) strain rat following prolonged oestrogen treatment. The cells are of the pre-T cell origin and their proliferation is dependent on mammalian lactogens, such as prolactin. Nb2-11 can also be mitogenically stimulated by IL-2. Injection of Nb2 cells into Nb rats gives rise to malignant tumors that are highly sensitive to treatment with vinca alkaloids. Karyotypic analysis has shown that the cell line has only five well developed chromosome abnormalities. The cells do not express surface immunoglobulin, and their lactogens dependency is confirmed. Protocols for the use of Nb2-11 cells in bioassays are available from ECACC on request.

As W02006102659 describes on page 74 paragraph 0240 example 7, the biological activity of rhGH and the conjugates described herein shall be assessed *in vitro* using an NB2-II rat lymphoma cell proliferation assay. Briefly, NB2-II cells derived from a rat lymphoma are incubated with rhGH, which lead to binding of the rhGH molecule to its receptor on the cell surface. Receptor binding induces the signal transduction cascade, which results in proliferation of the cells. Assay results are based on determined protein content, and a 100% bioactivity of unmodified rhGH.

### Conclusion:

Based on detailed instructions of this example 1 it is routine work for the skilled person to measure this residual activity of the prodrug.

### Example 2: Assay to measure autocleavage rate of the transient linker of the prodrug.

### Determination of in vitro autocleavage rate

For determination of *in vitro* linker cleavage rate of PEG-linker-rhGH conjugates, the compounds are dissolved in buffer at pH 7.4 (e.g. 10 mM sodium phosphate, 140 mM NaCl, 3 mM EDTA) and solution is filtered through a 0.22 µm filter and incubated at 37 °C. Samples are taken at time intervals and analyzed by RP-HPLC or size exclusion chromatography at 215 nm. UV-signals correlating to liberated rhGH are integrated and plotted against incubation time. Curve fitting software is applied to determine first- order cleavage rates.

### Linker cleavage rates in vivo and in vitro/in vivo correlation

Linker cleavage rates *in vivo* are determined by comparing the pharmacokinetics of permanent PEG-rhGH conjugates with the respective transient PEG-linker-rhGH conjugate carrying the same PEG moiety after intravenous injection into rat.

Firstly, permanent PEG-rhGH is injected intravenously into rats and blood samples are taken at time intervals, plasma prepared, and analyzed for rhGH using an ELISA.

Secondly, transient PEG-linker-rhGH is injected intravenously in rats, blood samples are taken at time intervals, plasma prepared, and analyzed for rhGH using an ELISA. Linker autocleavage half-life is calculated from the ratio of rhGH concentration of transient conjugate divided by determined rhGH concentration of permanent conjugate at the respective time points and curve fitting. Data are compared to *in vitro* cleavage rates.

### Conclusion

Based on detailed instructions of this example 2 it is routine work for the skilled person to measure the *in vivo* autocleavage rate of the transient linker of the prodrug.

### Example 3: Assay to measure Lipoatrophy

As said above compound PHA-794428 is a PEGylated-rhGH and described in patent EP1715887 from the company Pharmacia. According to www.clinicaltrials.gov, the study was terminated on 10-Dec-2007. Pfizer's (Pharmacia) decision to terminate the program was due to cases of injection-site lipoatrophy that were reported in the clinical Phase 2 studies after a single injection of PHA 794428. Lipoatrophy is the term describing the localized loss of fat tissue and is visible on humans as holes in the skin (visible by the eye).

### Assay

There are several in vitro methods described in the art in measure lipoatrophy. One proposal is described in publication J. Anim. Sci (1972), 35: 794-800 (L.J. Machlin) on page 795. Another description is found in Int. J. Cancer; 80, 444 - 447 (1999).

Generally, lipoatrophy can be measured as proposed below.

Lipolytic effect can be determined using an *in vitro* assay consisting of isolated mammal adipocytes, preferable murine adipocytes. Samples to be assayed were incubated at physiologically relevant conditions with a predetermined number of adipocytes in Krebs-Ringer bicarbonate buffer containing appropriate nutrients for up to 6 hours. The concentration of released glycerol is determined by standard methods, for example enzymatically or by a radiometric detection. Control samples containing adipocytes alone are analyzed to determine the spontaneous glycerol release.

The lipolytic effect of native unmodified recombinant human growth hormone and permanently PEGylated recombinant human growth hormone is compared to that of transiently PEGylated recombinant human growth hormone.

### Conclusion

Based on detailed instructions of this example 3 it is routine work for the skilled person to measure the lipoatrophy effect.

### REFERENCE LIST

1. Büyükgebiz A. et al J. Pediatr. Endocrinol. Metab. 1999 Jan-Feb; 12(1):95-7
2. Clark et al, 1996, Journal of Biological Chemistry 271: 21969-21977
3. Girard, J. Mehls, O., J. Clin Invest. 1994 March; 93(3): 1163-1171
4. Philip Harris et al. Horm. Res. 2006; 65 (suppl. 4): 1-213, CF1-98 GH/IGF Treatment with title "First in-human study of PEGylated recombinant human growth hormone".
5. Veronese, F.M. "Enzymes for Human Therapy: Surface Structure Modifications," Chimica Oggi, 7:53-56 (1989).

## Claims

1. A pharmaceutical composition comprising suitable pharmaceutical excipients and also comprising a human *in vivo* clinical effective amount of a recombinant human growth hormone (rhGH) PEGylated prodrug conjugate; wherein the prodrug is **characterized by** that:
(1): when PEG is linked to rhGH in the conjugate the conjugate has a GH activity with is less than 5% of the native growth hormone without PEG, measured according to the assay to measure rhGH PEGylated prodrug and rhGH activity of example 1; and
(2): PEG is linked to rhGH via a self hydrolysable (autocleavage) transient linker, wherein the linker autohydrolysis rate is such that the *in vivo* half-life is from 10 hours to 600 hours, measured according to the assay to measure autocleavage rate of the transient linker of the prodrug of example 2.

2. The pharmaceutical composition of claim 1, wherein the composition is a composition for subcutaneous administration, intramuscular administration or intravenous injection.

3. The pharmaceutical composition of claims 1 or 2, wherein the PEGylated prodrug has a total PEG load per growth hormone molecule that amounts to at least 25 kDa.

4. The pharmaceutical composition of any of claims 1 to 3, wherein the recombinant human growth hormone (rhGH) PEGylated prodrug has a lipoatrophy effect of less than 5% of the corresponding rhGH without PEG, measured according to the assay to determine lipoatrophy of example 3 and other identical dosage regimen conditions.

5. The pharmaceutical composition of any of the preceding claims, wherein the self hydrolysable transient linker comprises the chemical structure: 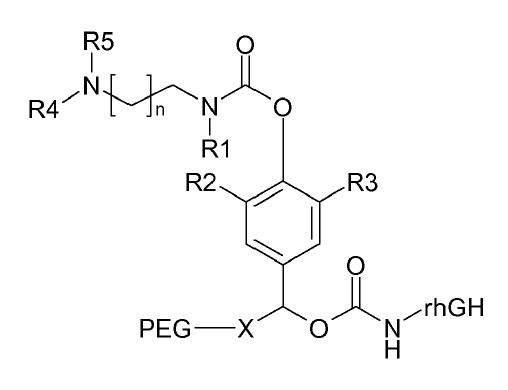 R1, R2, R3, R4, and R5 are selected independently from hydrogen, methyl, ethyl, propyl, isopropyl, butyl, isobutyl, tertiary butyl,
and n = 1 or 2, and
X is selected from C1 to C8 alkyl or C1 to C12 heteroalkyl.

6. The pharmaceutical composition of claim 5, wherein the formula I, which is a part of the structure within the general aromatic linker structure of claim 5, is 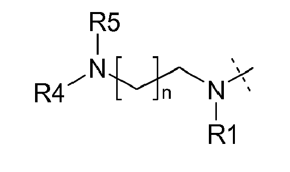 and formula I is selected from the group consisting of:

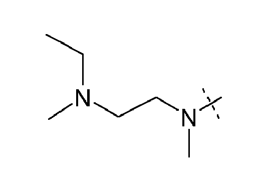

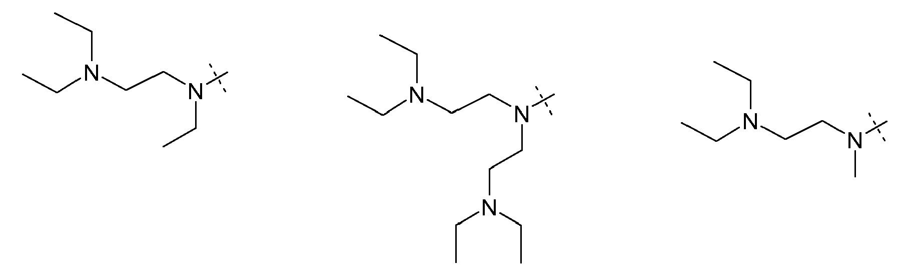

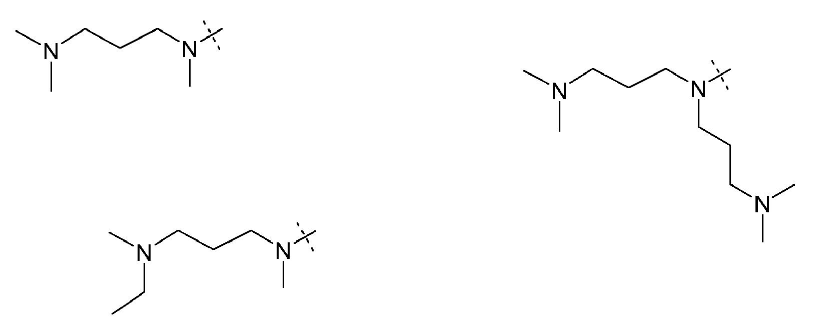

7. The pharmaceutical composition of claim 5, wherein the aromatic core, which is a part of the structure within the general aromatic linker structure of claim 5, has the general formula II: 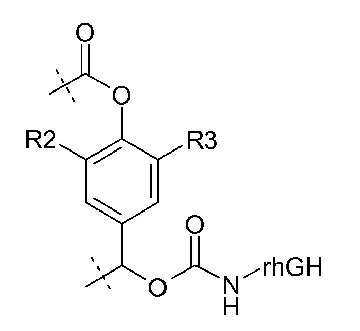 Formula II and formula II is selected from the group consisting of:

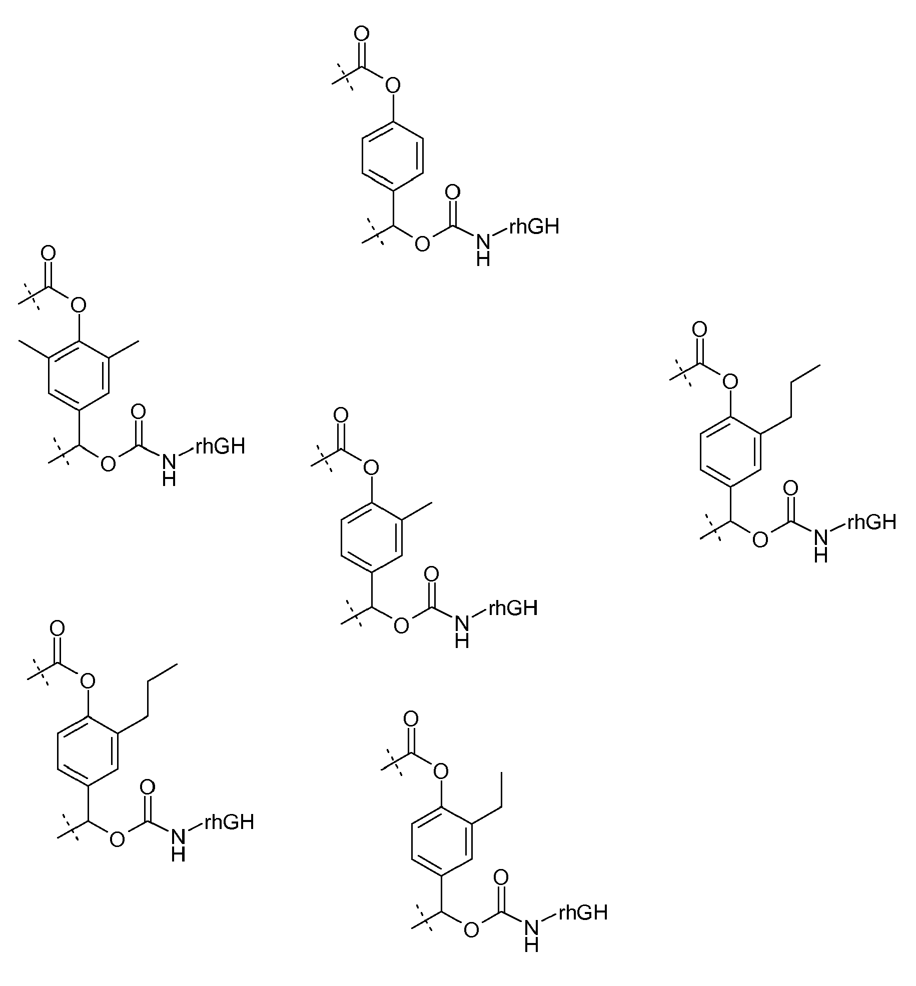

8. The pharmaceutical composition of claim 5, wherein the PEG W, which is a part of the structure within the general aromatic linker structure of claim 5, has the general formula III: 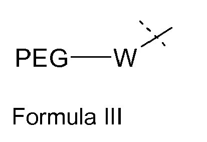 and PEG-W includes the following substituent groups:

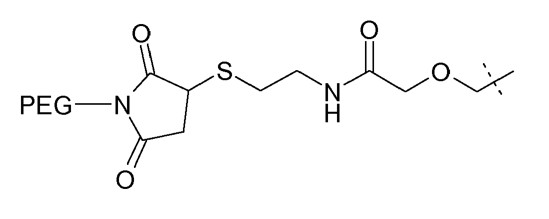

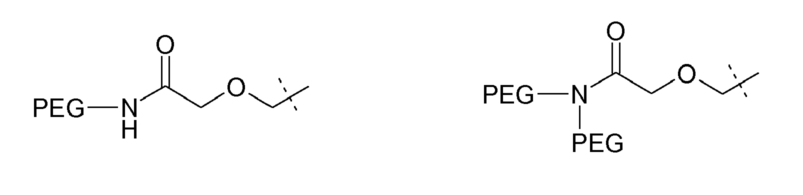

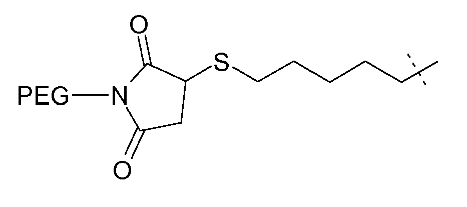

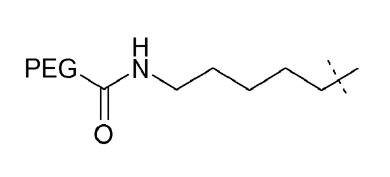

9. The pharmaceutical composition of any of claims 1 to 4, wherein the self hydrolysable transient linker structure is:

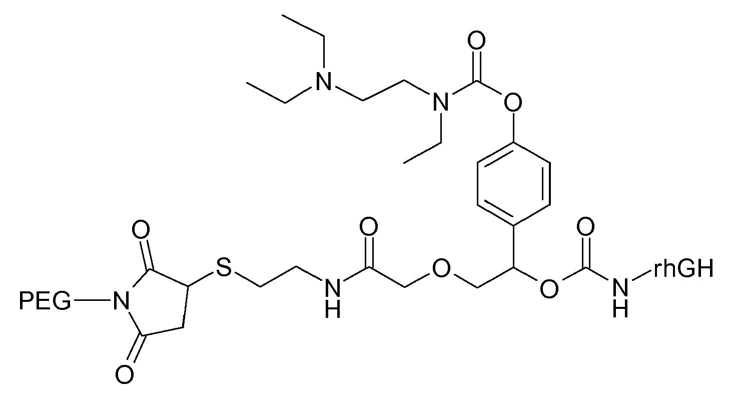

10. The pharmaceutical composition of any of claims 1 to 4, wherein the self hydrolysable transient linker structure is: 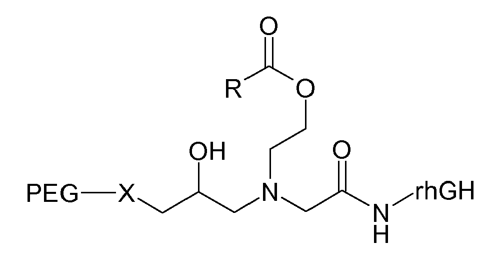 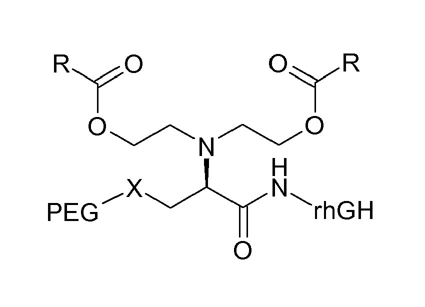 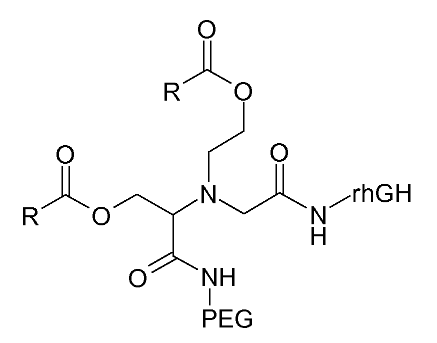 R is selected from hydrogen, methyl, ethyl, propyl and butyl.
X is selected from C1 to C8 alkyl or C1 to C12 heteroalkyl.

11. The pharmaceutical composition of any of the preceding claims, wherein the linker autocleavage rate *in vivo* is such that the *in vivo* half-life is from 50 to 75 hours.

12. A clinical effective amount of the pharmaceutical composition comprising the rhGH PEGylated prodrug of any of claims 1 to 11 for use in a method for treatment of a GH related disease in a human person.
